# EUROPEAN PATENT SPECIFICATION

(11) **EP 2 318 027 B1**
(45) Date of publication and mention of the grant of the patent: **02.02.2022**
(21) Application number: 09774420.5
(22) Date of filing: 01.07.2009
(51) Int. Cl.: C12P 17/10, C12P 17/18, C07D 211/42, C07D 491/048, C07H 15/256, C07J 61/00

(54) **ISOLATION OF DEGLYCOSYLATED VERATRUM ALKALOIDS**
ISOLIERUNG VON DEGLYCOSYLIERTEN VERATRUM ALKALOIDEN
ISOLEMENT DES ALCALOÏDES DÉGLYCOSYLÉS DE VERATRUM

(30) Priority: 02.07.2008 US 77703 P
(43) Date of publication of application: 11.05.2011
(73) Proprietor: Infinity Pharmaceuticals, Inc., Cambridge, MA 02139 (US)
(72) Inventor: JAYATILAKE, Gamini, Senerath, Broomfield CO 80020 (US); RICHHEIMER, Steven, L., Steamboat Springs, CO 80487 (US); MANN, David, A., Madison WI 53717 (US)
(74) Representative: HGF
(86) International application number: PCT/US2009/049372
(87) International publication number: WO 2010/002970

(56) References cited:
- EP-A2- 0 255 331
- WO-A1-2010/000070
- US-A1- 2005 203 061
- TANG, J. ET AL.: "Antitumor Activity of Extracts and Compounds from the Rhizomes of Veratrum dahuricum", PHYTOTHERAPY RESEARCH, vol. 22, 20 June 2008 (2008-06-20), pages 1093-1096, XP002761189,
- CONG, Y. ET AL.: "Steroidal Alkaloids from the Roots and Rhizomes of Vertrum nigrum L.", HELVETICA CHIMICA ACTA, vol. 90, no. 5, 2007, pages 1038-1042, XP002761207,
- KEELER, R.F.: "TRERATOGENIC COMPOUNDS OF VERATRUM CALIFORNICUM (DURAND). VII. THE STRUCTURE OF THE GLYCOSIDIC ALKALOID CYCLOPOSINE", STEROIDS, vol. 13, no. 5, 1969, pages 579-588, XP002761229,
- KEELER, R.F. & BINNS, W.: "TERATOGENIC COMPOUNDS OF VERATRUM CALIFORNICUM (DURAND). I. PREPARATION AND CHARACTERIZATION OF FRACTIONS AND ALKALOIDS FOR BIOLOGIC TESTING", CANADIAN JOURNAL OF BIOCHEMISTRY, vol. 44, no. 6, June 1966 (1966-06), pages 819-828, XP008142873,
- KEEKER R. F.: 'Teratogenic compounds of Veratrum californicum (Durand). VII. The structure of the glycosidic alkaloid cycloposine' STEROIDS vol. 13, no. 5, May 1969, pages 579 - 588, XP023444238
- KEEKER R. F. ET AL: 'Teratogenic compounds of Veratrum californicum (Durand). I. Preparation and characterization of fractions and alkaloids for biologic testing' CANADIAN JOURNAL OF BIOCHEMISTRY vol. 44, no. 6, June 1966, pages 819 - 828, XP008142873

## Description

### BACKGROUND

In the mid-1950's the births of severely deformed one-eyed lambs were reported in central Idaho. The US Department of Agriculture and the FDA, over the course of an 11-year study, determined that maternal ingestion of the wild corn lily *Veratrum californicum* by pregnant ewes on day 14 of gestation induced this cyclopian-type malformation in the lambs. *V. californicum* grows on open sub-alpine meadows and hillsides of western United States at elevations of between 5,000 to 11,000 feet. The main teratogenic compound in *V. californicum* that induces the cyclopian-type deformity was subsequently isolated and indentified as 11-deoxojervine, dubbed "cyclopamine." Cyclopamine acts as a hedgehog (Hh) pathway inhibitor, blocking the function of the Sonic hedgehog gene essential for embryonic development (Cooper et al., Science (1998) 280: 1603-1607; Chen et al., Genes and Development (2002) 16: 2743-2748; Incardona et al., Dev. Biol. (2000) 224:440; and Chen et al. Proc. Natl. Acad. Sci. USA (2002) 99:14071). Other *Veratrum* alkaloids which may be present in *Veratrum californicum* include, but are not limited to, cycloposine, veratramine, veratrosine, jervine and muldamine.

Cyclopamine, despite its teratogenic nature, is a potent anti-cancer agent. In studies using cyclopamine, researchers have stopped the growth of the most virulent human tumors, varieties accounting for 25% of cancer deaths. Cyclopamine and cyclopamine analogs are currently being investigated as treatment agents in several different cancers, such as, for example, basal cell carcinoma, medulloblastoma, rhabdomyosarcoma, lung cancer, pancreatic cancer, breast cancer, glioblastoma, and as a treatment agent for multiple myeloma.

A method for the isolation of cyclopamine from *V. californicum* was described several years ago by Keeler and co-workers (Keeler, Phytochemistry (1968) 7:303-306), but the method produced only milligram quantities of cyclopamine from kilogram quantities of dried plant material. Thus, there continues to be a need for new and improved isolation methods of cyclopamine.

### SUMMARY

A significant amount of *Veratrum* alkaloids are stored in *Veratrum californicum* as glycosylated derivatives. Provided are novel deglycosylation methods designed to optimize the yield of the deglycosylated *Veratrum* alkaloid from *Veratrum* plant material and/or from an extract of *Veratrum* plant material.

Provided is a method of isolating a deglycosylated *Veratrum* alkaloid from *Veratrum* plant material, comprising the steps of:
(i) providing a *Veratrum* plant material comprising a glycosylated *Veratrum* alkaloid,
(ii) prior to an extracting step, contacting the *Veratrum* plant material with an aqueous solution at a pH between 5 and 7.5, the aqueous solution comprising greater than 25% water, and
(iii) extracting the *Veratrum* plant material with a solvent to provide an extract comprising the deglycosylated *Veratrum* alkaloid.

The invention is as defined in the appended claims.

### BRIEF DESCRIPTION OF THE DRAWINGS

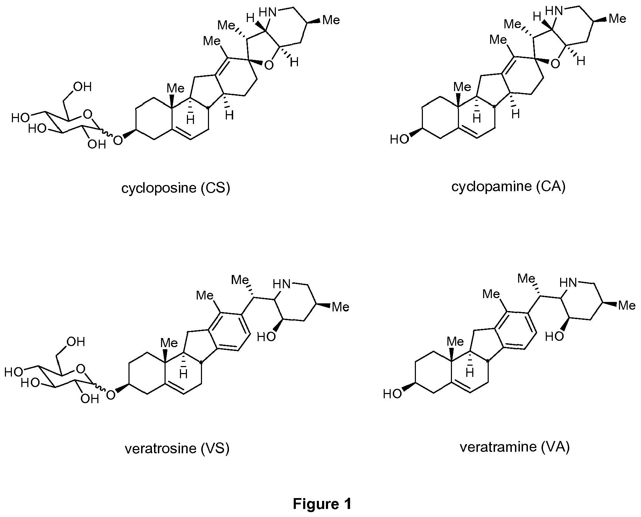

**FIGURE 1** depicts the chemical structures of cycloposine (CS), cyclopamine (CA), veratrosine (VS) and veratramine (VA).
**FIGURE 2** depicts the amount (in g/kg) of cyclopamine present in different biomass samples of *Veratrum californicum.*
**FIGURE 3** depicts the amount (in g/kg) of cyclopamine (CA) and cycloposine (CS) present in different biomass samples of *Veratrum californicum.*
**FIGURE 4** depicts the enzymatic conversion of purified cycloposine to cyclopamine via deglycosidation using β-glucuronidase (*Helix pomatia*) in acetate buffer (pH 5.1) as monitored by liquid chromatography-mass spectrometry (LCMS).
**FIGURE 5** depicts HPLC traces comparing the efficiency of the enzymatic conversion of purified cycloposine to cyclopamine using β-glucuronidase (*Helix pomatia*) in acetate buffer (pH 5.1) and Tris buffer (pH 7.2).
**FIGURE 6** depicts an LCMS trace showing peaks corresponding to cycloposine, vertatrosine, cyclopamine and veratramine in a crude *Veratrum* extract treated with β-glucuronidase (*Helix pomatia*)*.*
**FIGURE 7** depicts the effect of enzyme concentration on the conversion of cycloposine to cyclopamine.
**FIGURE 8** depicts the conversion of glycosylated alkaloids in a crude *Veratrum* extract upon treatment with water, followed by MeOH extraction.
**FIGURE 9** depicts the conversion of cycloposine to cyclopamine from various biomass samples.
**FIGURE 10** depicts the conversion of glycosylated alkaloids in a crude *Veratrum* extract upon treatment and extraction with either MeOH or 50% MeOH in water.

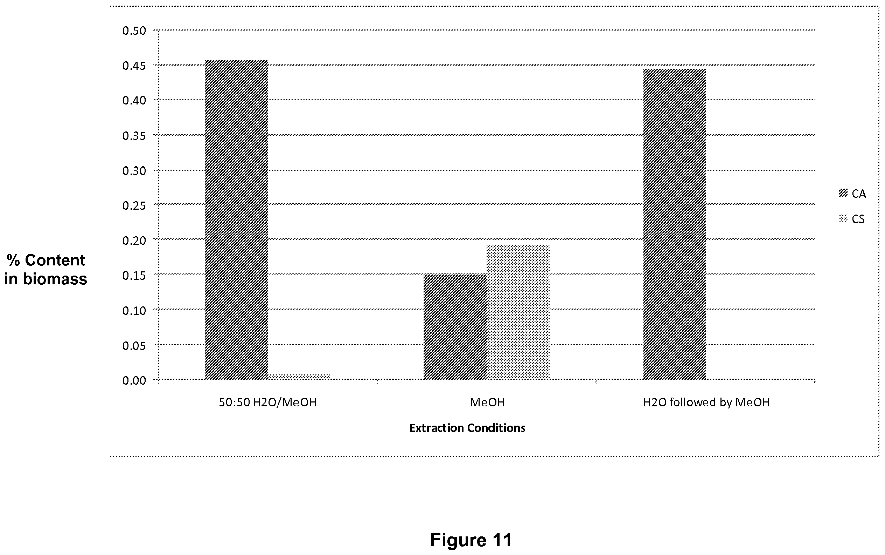

**FIGURE 11** compares the results of treating the biomass under various conditions: (i) treatment with 50% MeOH in water, followed by extraction as described in Figure 10; (ii) treatment with 100% MeOH, followed by extraction as described in Figure 10; and (iii) treatment with water followed by extraction with MeOH, as described in Figure 8.

### DETAILED DESCRIPTION

Provided are inventive deglycosylation methods designed to optimize the yield of deglycosylated *Veratrum* alkaloids from *Veratrum* plant material.

### Aqueous Pre-treatment Method

Significant amounts of *Veratrum* alkaloids, such as cyclopamine and veratramine, exist in *Veratrum californicum* as their respective glycosylated derivatives (*e.g.,* for example, cycloposine and veratrosine). It is generally believed that one or more endogenous enzymes present in the *Vertraum* plant facilitate the transformation of these Veratrum alkaloids to and from their glycosylated derivatives depending upon the requirements of the plant.

It has been discovered that, upon treatment of harvested and dried *Veratrum* plant material with an aqueous solution prior to extraction, the amount of glycosylated *Veratrum* alkaloids decreased, and upon extraction the isolated yield of deglycosylated *Veratrum* alkaloids increased. Without wishing to be bound by any theory, it is proposed that during aqueous treatment one or more endogenous enzymes present in the *Veratrum* plant material facilitate deglycosylation of glycosylated *Veratrum* alkaloids, thereby increasing the observed isolated yield of the deglycosylated *Veratrum* alkaloids.

Thus, provided is a method of isolating a deglycosylated *Veratrum* alkaloid from *Veratrum* plant material, comprising the steps of:
(i) providing a *Veratrum* plant material comprising a glycosylated *Veratrum* alkaloid;
(ii) contacting the *Veratrum* plant material with an aqueous solution; and
(iii) extracting the *Veratrum* plant material with a solvent to provide an extract comprising the deglycosylated *Veratrum* alkaloid.

In certain embodiments, the glycosylated *Veratrum* alkaloid is cycloposine, and the deglycosylated *Veratrum* alkaloid is cyclopamine.

In certain embodiments, the *Veratrum* plant material comprises a mixture of cycloposine and cyclopamine.

In certain embodiments, the glycosylated *Veratrum* alkaloid is veratrosine, and the deglycosylated *Veratrum* alkaloid is veratramine.

In certain embodiments, the *Veratrum* plant material comprises a mixture of veratrosine and veratramine.

In certain embodiments, the *Veratrum* plant material comprises a mixture of cycloposine and veratrosine.

In certain embodiments, the *Veratrum* plant material comprises a mixture of cycloposine, cyclopamine, veratrosine and veratramine.

As used herein, "*Veratrum* plant material" refers to harvested plants of *Veratrum californicum*, such as *Veratrum californicum* var. *californicum*, which may be optionally dried and optionally ground into a fine powder. *Veratrum* plant material may also comprise harvested plants genetically engineered to produce or contain one or more *Veratrum* alkaloids (*e.g.,* a plant genetically engineered to produce high levels of one or more *Veratrum* alkaloids, such as cyclopamine and/or cycloposine).

In certain embodiments, the pH of the aqueous solution is at or below a pH of about 9. In certain embodiments, the pH of the aqueous solution is at or below a pH of about 8. In certain embodiments, the pH of the aqueous solution is at or below a pH of about 7.5. In certain embodiments, the pH of the aqueous solution is at or below a pH of about 7.

In certain embodiments, the pH of the aqueous solution is between 5 to about 7, inclusive. In certain embodiments, the pH of the aqueous solution is between 5 to about 6, inclusive. In certain embodiments, the pH of the aqueous solution is between about 6 to 7.5, inclusive.

In certain embodiments, the aqueous solution is neutral (*i.e.,* at a pH of about 7).

In certain embodiments, the aqueous solution is acidic (*i.e.,* below a pH of about 7).

In certain embodiments, the aqueous solution does not comprise a base.

In certain embodiments, the aqueous solution does not comprise an inorganic base. In certain embodiments, the aqueous solution does not comprise an organic base.

In certain embodiments, the aqueous solution does not comprise ammonium hydroxide or sodium carbonate. In certain embodiments, the aqueous solution does not comprise ammonium hydroxide.

In certain embodiments, the aqueous solution is buffered. Exemplary buffers include, but are not limited to, 3-{[tris(hydroxymethyl)methyl]amino}propanesulfonic acid (TAPS), N,N-bis(2-hydroxyethyl)glycine (Bicine), tris(hydroxymethyl)methylamine (Tris), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES),3-(N-morpholino)propanesulfonic acid (MOPS), piperazine-N,N'-bis(2-ethanesulfonic acid) (PIPES), dimethylarsinic acid (Cacodylate), 2-(N-morpholino)ethanesulfonic acid (MES), carbonic acid buffer, phosphate buffered saline (PBS), acetate buffer, and salts thereof. In certain embodiments, the buffer is acetate buffer. In certain embodiments, the buffer is Tris buffer.

In certain embodiments, the aqueous solution comprises greater than 25%, greater than about 30%, greater than about 35%, greater than about 40%, greater than about 45%, greater than about 50%, greater than about 60%, greater than about 65%, greater than about 70%, greater than about 75%, greater than about 80%, greater than about 85%, greater than about 90%, greater than about 95%, greater than about 98% or greater than about 99% water.

In certain embodiments, the aqueous solution comprises between about 5% to about 100% water, inclusive. In certain embodiments, the aqueous solution comprises between about 10% to about 100% water, inclusive. In certain embodiments, the aqueous solution comprises between about 20% to about 100% water, inclusive. In certain embodiments, the aqueous solution comprises between about 30% to about 100% water, inclusive. In certain embodiments, the aqueous solution comprises between about 40% to about 100% water, inclusive. In certain embodiments, the aqueous solution comprises between about 50% to about 100% water, inclusive. In certain embodiments, the aqueous solution comprises between about 60% to about 100% water, inclusive. In certain embodiments, the aqueous solution comprises between about 70% to about 100% water, inclusive. In certain embodiments, the aqueous solution comprises between about 80% to about 100% water, inclusive. In certain embodiments, the aqueous solution comprises between about 90% to about 100% water, inclusive.

In certain embodiments, the aqueous solution comprises a mixture of water and a co-solvent. Exemplary co-solvents include organic alcohols, such as methanol, ethanol and isopropanol. In certain embodiments, the aqueous solution is a mixture of water and methanol.

In certain embodiments, the aqueous solution comprises about a 1:1 (v/v), about a 1:2 (v/v), about a 1:3 (v/v), about a 1:4 (v/v), about a 1:5 (v/v), about a 1:6 (v/v), about a 1:7 (v/v), about a 1:8 (v/v), about a 1:9 (v/v), or about a 1:10 (v/v) mixture of water and a co-solvent.

In certain embodiments, the aqueous solution comprises about a 1:1 (v/v), about a 1:2 (v/v), about a 1:3 (v/v), about a 1:4 (v/v), about a 1:5 (v/v), about a 1:6 (v/v), about a 1:7 (v/v), about a 1:8 (v/v), about a 1:9 (v/v), or about a 1:10 (v/v) mixture of a co-solvent and water.

In certain embodiments, the aqueous solution is 100% water.

In certain embodiments, the total weight of the aqueous solution is at least about 1.5 times (w/w), at least about 2 times (w/w), at least about 3 times (w/w), at least about 4 times (w/w) or at least about 5 times (w/w), the weight of the *Veratrum* plant material.

In certain embodiments, the aqueous solution is at a temperature of below about 100 °C, below about 90 °C, below about 80 °C, below about 70 °C, below about 60 °C, below about 50 °C, below about 40 °C, below about 30 °C, below about 28 °C, below about 25 °C, below about 20 °C, below about 15 °C, below about 10 °C or below about 5 °C.

In certain embodiments, the aqueous solution is at a temperature of between about 0 °C to about 100 °C, inclusive. In certain embodiments, the aqueous solution is at a temperature of between about 0 °C to about 90°C, inclusive. In certain embodiments, the aqueous solution is at a temperature of between about 0 °C to about 80°C, inclusive. In certain embodiments, the aqueous solution is at a temperature of between about 0 °C to about 70°C, inclusive. In certain embodiments, the aqueous solution is at a temperature of between about 0 °C to about 60°C, inclusive. In certain embodiments, the aqueous solution is at a temperature of between about 0 °C to about 50°C, inclusive. In certain embodiments, the aqueous solution is at a temperature of between about 0 °C to about 40°C, inclusive. In certain embodiments, the aqueous solution is at a temperature of between about 0 °C to about 30°C, inclusive. In certain embodiments, the aqueous solution is at a temperature of between about 0 °C to about 25°C, inclusive. In certain embodiments, the aqueous solution is at a temperature of between about 25 °C to about 75°C, inclusive. In certain embodiments, the aqueous solution is at a temperature of between about 30 °C to about 60°C, inclusive.

In certain embodiments, during said contacting step, the amount of glycosylated *Veratrum* alkaloid decreases. In certain embodiments, during said contacting step, the amount of deglycosylated *Veratrum* alkaloid increases. In certain embodiments, during said contacting step, the amount of glycosylated *Veratrum* alkaloid decreases and the amount of deglycosylated *Veratrum* alkaloid increases.

In certain embodiments, during said contacting step, the glycosylated *Veratrum* alkaloid is converted to deglycosylated *Veratrum* alkaloid by one or more endogenous enzymes present in the plant material.

In certain embodiments, the contacting step comprises contacting the *Veratrum* plant material with an aqueous solution for a period of time sufficient to convert approximately 10% of the glycosylated *Veratrum* alkaloid present in the plant material to the deglycosylated *Veratrum* alkaloid. In certain embodiments, the contacting step comprises contacting the *Veratrum* plant material with an aqueous solution for a period of time sufficient to convert at least about 25% of the glycosylated *Veratrum* alkaloid present in the plant material to the deglycosylated *Veratrum* alkaloid. In certain embodiments, the contacting step comprises contacting the *Veratrum* plant material with an aqueous solution for a period of time sufficient to convert at least about 50% of the glycosylated *Veratrum* alkaloid present in the plant material to the deglycosylated *Veratrum* alkaloid. In certain embodiments, the contacting step comprises contacting the *Veratrum* plant material with an aqueous solution for a period of time sufficient to convert at least about 75% of the glycosylated *Veratrum* alkaloid present in the plant material to the deglycosylated *Veratrum* alkaloid. In certain embodiments, the contacting step comprises contacting the *Veratrum* plant material with an aqueous solution for a period of time sufficient to convert at least about 95% of the glycosylated *Veratrum* alkaloid present in the plant material to the deglycosylated *Veratrum* alkaloid.

In certain embodiments, all of the glycosylated *Veratrum* alkaloid present in the plant material is converted to the deglycosylated *Veratrum* alkaloid.

In certain embodiments, the contacting step comprises contacting the *Veratrum* plant material with an aqueous solution for at least 5 minutes. In certain embodiments, the contacting step comprises contacting the *Veratrum* plant material with an aqueous solution for at least 10 minutes. In certain embodiments, the *Veratrum* plant material is contacted with an aqueous solution for at least 30 minutes. In certain embodiments, the *Veratrum* plant material is contacted with an aqueous solution for at least 45 minutes. In certain embodiments, the *Veratrum* plant material is contacted with an aqueous solution for at least 1 hour. In certain embodiments, the *Veratrum* plant material is contacted with an aqueous solution for at least 1.5 hours. In certain embodiments, the *Veratrum* plant material is contacted with an aqueous solution for at least 2 hours.

In certain embodiments, the contacting step comprises contacting the *Veratrum* plant material with an aqueous solution for between about 5 minutes to about 5 hours, inclusive. In certain embodiments, the contacting step comprises contacting the *Veratrum* plant material with an aqueous solution for between about 5 minutes to about 2 hours, inclusive. In certain embodiments, the contacting step comprises contacting the *Veratrum* plant material with an aqueous solution for between about 5 minutes to about 1 hour, inclusive.

In certain embodiments, the contacting step comprises agitating the *Veratrum* plant material in an aqueous solution. In certain embodiments, the step of agitating comprises shaking or stirring the plant material in the aqueous solution. In other embodiments, the contacting step does not comprise agitation.

In certain embodiments, the method further comprises the step of removing the *Veratrum* plant material from the aqueous solution prior to extraction of the *Veratrum* plant material. In certain embodiments, the step of removing comprises filtration or centrifugation.

In certain embodiments, after the first contacting step but prior to the extracting step, the method further comprises contacting the plant material with a basic aqueous solution.

In certain embodiments, the extracting step comprises extracting the *Veratrum* plant material with a solvent to provide an extract comprising the deglycosylated *Veratrum* alkaloid, wherein the solvent comprises one or more organic solvents optionally mixed with water and/or a base.

In certain embodiments, the organic solvent is an organic alcohol, an ester, a ketone, an ether, a halogenated hydrocarbon, a hydrocarbon, an aromatic, or a heteroaromatic, or a mixture of two or more thereof.

Exemplary organic alcohols include, but are not limited to, methanol, ethanol, propanol, isopropanol, 2-butanol and n-butanol. Exemplary esters include, but are not limited to, ethyl acetate and isopropyl acetate. Exemplary ketones include, but are not limited to, acetone and methyl ethyl ketone (MEK). Exemplary ethers include, but are not limited to, tetrahydrofuran (THF), dioxane and diethyl ether. Exemplary halogenated hydrocarbons include, but are not limited to, dichloromethane, dichloroethane and chloroform. Exemplary hydrocarbons include, but are not limited to, hexanes, heptanes and pentanes. Exemplary aromatic solvents include, but are not limited to, benzene, anisole, toluene and xylenes.

In certain embodiments, the organic solvent is an organic alcohol. In certain embodiments, the organic solvent is methanol.

In certain embodiments, the solvent used in the extracting step comprises a mixture of an organic solvent and water. In some embodiments, the solvent used in the extracting step comprises a mixture of methanol and water.

In certain embodiments, the solvent used in the extraction step comprises a mixture of about a 1:1 (v/v), about a 1:2 (v/v), about a 1:3 (v/v), about a 1:4 (v/v), about a 1:5 (v/v), about a 1:6 (v/v), about a 1:7 (v/v), about a 1:8 (v/v), about a 1:9 (v/v), or about a 1:10 (v/v) mixture of water and organic solvent.

In certain embodiments, the solvent used in the extraction step comprises a mixture of about a 1:1 (v/v), about a 1:2 (v/v), about a 1:3 (v/v), about a 1:4 (v/v), about a 1:5 (v/v), about a 1:6 (v/v), about a 1:7 (v/v), about a 1:8 (v/v), about a 1:9 (v/v), or about a 1:10 (v/v) mixture of organic solvent and water.

In certain embodiments, the solvent used in the extraction step comprises a mixture of an organic solvent and a base. In some embodiments, the base is an aqueous basic solution. In other embodiments, the base is non-aqueous.

In certain embodiments, the base is present in the organic solvent in less than about 20% (v/v), less than about 15% (v/v), less than about 10% (v/v), less than about 5% (v/v) or less than about 1% (v/v). In certain embodiments, the base is present in the organic solvent in between about 1% to about 20% (v/v), inclusive; between about 1% to about 10% (v/v), inclusive; between about 1% to about 10% (v/v), inclusive or between about 1% to about 5% (v/v), inclusive.

Exemplary bases include organic bases and inorganic bases. Exemplary inorganic bases include, but are not limited to, aqueous solutions of ammonium hydroxide, sodium hydroxide, sodium carbonate, sodium bicarbonate, sodium acetate, sodium citrate, potassium hydroxide, potassium carbonate, potassium bicarbonate, potassium sodium tartrate (*aka* Rochelle's salt), and lithium hydroxide. Exemplary organic bases include, but are not limited to, triethylamine, diethylisopropyl amine and pyridine. In certain embodiments, the base is an aqueous solution of sodium carbonate. In certain embodiments, the base is triethylamine. In certain embodiments, the base is an aqueous solution of sodium hydroxide.

In some embodiments, the solvent used in the extracting step comprises a mixture of methanol and an aqueous solution of sodium carbonate. In other embodiments, the solvent used in the extracting step comprises a mixture of methanol and triethylamine. In still other embodiments, the solvent used in the extracting step comprises a mixture of methanol and an aqueous solution of sodium hydroxide. In certain embodiments, the method further comprises the step of concentrating the solvent after the step of extraction to provide an extract comprising deglycosylated *Veratrum* alkaloid.

In certain embodiments, the method further comprises purifying deglycosylated *Veratrum* alkaloid isolated from the extract. In certain embodiments, the step of purifying comprises providing deglycosylated *Veratrum* alkaloid having greater than about 85% purity. In certain embodiments, the step of purifying comprises providing deglycosylated *Veratrum* alkaloid having greater than about 90% purity. In certain embodiments, the step of purifying comprises providing deglycosylated *Veratrum* alkaloid having greater than about 92% purity. In certain embodiments, the step of purifying comprises providing deglycosylated *Veratrum* alkaloid having greater than about 95% purity.

Overall purity of an organic compound can be determined using various analytical techniques known in the art, such as, for example, liquid chromatography mass spectrometry (LC-MS) and high pressure liquid chromatography (HPLC). Other methods useful in the characterization and purity of an organic compound include melting point, optical rotation and nuclear magnetic resonance spectroscopy (NMR).

In certain embodiments, the step of purifying comprises chromatographic purification. In certain embodiments, the chromatographic purification comprises silica gel chromatographic purification.

In certain embodiments, the step of purifying comprises trituration.

In certain embodiments, the step of purifying comprises crystallization.

In certain embodiments, the present invention provides a method of isolating cyclopamine from *Veratrum* plant material, comprising the steps of:
(i) providing a *Veratrum* plant material comprising cycloposine;
(ii) contacting the *Veratrum* plant material with an aqueous solution; and
(iii) extracting the *Veratrum* plant material with a solvent to provide an extract comprising cyclopamine.

In certain embodiments, during said contacting step, the amount of cycloposine decreases. In certain embodiments, during said contacting step, the amount of cyclopamine increases. In certain embodiments, during said contacting step, the amount of cycloposine decreases and the amount of cyclopamine increases.

In certain embodiments, during said contacting step, cycloposine is converted to cyclopamine by one or more endogenous enzymes present in the plant material.

In certain embodiments, the pH of the aqueous solution is at or below a pH of about 8. In certain embodiments, the pH of the aqueous solution is between about 4 and about 8, inclusive.

In certain embodiments, the aqueous solution does not comprise a base. In certain embodiments, the aqueous solution does not comprise ammonium hydroxide or sodium carbonate.

In certain embodiments, the aqueous solution comprises greater than about 25% water. In certain embodiments, the aqueous solution comprises between about 30% to about 100% water, inclusive. In some embodiments, the aqueous solution comprises 100% water.

In certain embodiments, the aqueous solution comprises a mixture of water and a co-solvent. In certain embodiments, the co-solvent is methanol.

In certain embodiments, the method further comprises the step of removing the *Veratrum* plant material from the aqueous solution prior to extraction of the *Veratrum* plant material.

In certain embodiments, the solvent used in the extracting step comprises one or more organic solvents optionally mixed with water and/or a base. In certain embodiments, the organic solvent is methanol. In certain embodiments, the solvent used in the extraction step comprises a mixture of methanol mixed with water. In certain embodiments, the solvent used in the extraction step comprises a mixture of methanol mixed with an aqueous basic solution (e.g., an aqueous solution of sodium carbonate or an aqueous solution of sodium hydroxide). In other embodiments, the solvent used in the extraction step comprises a mixture of methanol mixed with an organic base (e.g., triethylamine).

### Exogenous Enzymatic Method

Further disclosed is a deglycosylation method comprising contacting a glycosylated *Veratrum* alkaloid and an enzyme in a buffered solution to provide a deglycosylated *Veratrum* alkaloid.

For example, the glycosylated *Veratrum* alkaloid is cycloposine, and the deglycosylated *Veratrum* alkaloid is cyclopamine.

For example, the glycosylated *Veratrum* alkaloid is veratrosine, and the deglycosylated *Veratrum* alkaloid is veratramine.

For example, the enzyme is an enzyme derived or isolated from a eukaryotic cell *(i.e.,* a eukaryotic-derived enzyme). In another example, the enzyme is an enzyme derived or isolated from a prokaryotic cell (*i.e.,* a prokaryotic-derived enzyme).

For example, the enzyme is a β-glucuronidase enzyme. In certain embodiments, the β-glucuronidase enzyme is selected from a *Helix pomatia* β-Glucuronidase enzyme, a *Helix aspersa* β-Glucuronidase enzyme, a *Patella vulgata* β-Glucuronidase enzyme, a Bovine Type 10 liver β-Glucuronidase enzyme, or a β-Glucuronidase enzyme derived from almonds. For example, the β-glucuronidase enzyme is a *Helix pomatia* β-glucuronidase enzyme.

For example, the pH of the buffered solution is at or below a pH of about 9. For example, the pH of the buffered solution is at or below a pH of about 8. For example, the pH of the buffered solution is at or below a pH of about 7.5. For example, the pH of the buffered solution is at or below a pH of about 7. For example, the pH of the buffered solution is at or below a pH of about 6. For example, the pH of the buffered solution is at or below a pH of about 5.5.

For example, the pH of the buffered solution is between about 4 to about 9, inclusive. For example, the pH of the buffered solution is between about 5 to about 8, inclusive. For example, the pH of the buffered solution is between about 5 to about 7.5, inclusive. For example, the pH of the buffered solution is about 5 to about 5.5, inclusive. For example, the pH of the buffered solution is between about 7 to about 7.5, inclusive.

Exemplary buffers include, but are not limited to, 3-{[tris(hydroxymethyl)methyl] amino}propanesulfonic acid (TAPS), N,N-bis(2-hydroxyethyl)glycine (Bicine), tris(hydroxymethyl)methylamine (Tris), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), 3-(N-morpholino)propanesulfonic acid (MOPS), piperazine-N,N'-bis(2-ethanesulfonic acid)

(PIPES), dimethylarsinic acid (Cacodylate), 2-(N-morpholino)ethanesulfonic acid (MES), carbonic acid buffer, phosphate buffered saline (PBS), acetate buffer, and salts thereof. For example, the buffer is acetate buffer. Exemplary, the buffer is Tris buffer.

For example, the temperature of the buffered solution is below about 100 °C, below about 90 °C, below about 80 °C, below about 70 °C, below about 60 °C, below about 50 °C or below about 40 °C.

For example, the temperature of the buffered solution is between about 25 °C to about 75 °C, inclusive. For example, the temperature of the buffered solution is between about 25 °C to about 50 °C, inclusive. For example, the temperature of the buffered solution is between about 25 °C to about 40 °C, inclusive. For example, the temperature of the buffered solution is at or about 37 °C.

For example, the contacting step comprises contacting the glycosylated *Veratrum* alkaloid with the enzyme for a period of time sufficient to convert approximately 10% of the glycosylated *Veratrum* alkaloid to the deglycosylated *Veratrum* alkaloid. For example, the contacting step comprises contacting the glycosylated *Veratrum* alkaloid with the enzyme for a period of time sufficient to convert at least about 25% of the glycosylated *Veratrum* alkaloid to the deglycosylated *Veratrum* alkaloid. For example, the contacting step comprises contacting the glycosylated *Veratrum* alkaloid with the enzyme for a period of time sufficient to convert at least about 50% of the glycosylated *Veratrum* alkaloid to the deglycosylated *Veratrum* alkaloid. For example, the contacting step comprises contacting the glycosylated *Veratrum* alkaloid with the enzyme for a period of time sufficient to convert at least about 75% of the glycosylated *Veratrum* alkaloid to the deglycosylated *Veratrum* alkaloid. For example, the contacting step comprises contacting the glycosylated *Veratrum* alkaloid with the enzyme for a period of time sufficient to convert at least about 95% of the glycosylated *Veratrum* alkaloid to the deglycosylated *Veratrum* alkaloid.

For example, the contacting step comprises contacting the glycosylated *Veratrum* alkaloid with the enzyme for a period of time sufficient to convert all of the glycosylated *Veratrum* alkaloid to the deglycosylated *Veratrum* alkaloid.

For example, the contacting step comprises contacting the glycosylated *Veratrum* alkaloid with the enzyme for at least 30 minutes. For example, the contacting step comprises contacting the glycosylated *Veratrum* alkaloid with the enzyme for at least 1 hour. For example, the contacting step comprises contacting the glycosylated *Veratrum* alkaloid with the enzyme for at least 2 hours. For example, the contacting step comprises contacting the glycosylated *Veratrum* alkaloid with the enzyme for at least 5 hours. For example, the contacting step comprises contacting the glycosylated *Veratrum* alkaloid with the enzyme for at least 10 hours. For example, the contacting step comprises contacting the glycosylated *Veratrum* alkaloid with the enzyme for at least 15 hours.

Further disclosed is a deglycosylation method comprising contacting cycloposine and a β-glucuronidase eukaryotic-derived enzyme in a buffered solution to provide cyclopamine.

For example, the enzyme is a *Helix pomatia* β-glucuronidase eukaryotic-derived enzyme.

### EXEMPLIFICATION

A series of studies was designed to investigate whether cycloposine could be converted to cyclopamine via enzymatic deglyclosylation. These studies showed not only that cycloposine could be enzymatically converted to cyclopamine in crude extraction mixtures, but so too could veratrosine be converted to veratramine. The enzymatic conversion was demonstrated using different enzymes from a variety of sources.

It was also discovered that the conversion of cycloposine to cyclopamine, as well as veratrosine to veratramine, can be effected by treatment of the harvested *Veratrum* plant material with an aqueous solution prior to extraction. The prior exogenous enzymatic studies support the hypothesis that one or more endogenous enzymes present in the *Veratrum* plant material may be effecting conversion of cycloposine to cyclopamine and veratrosine to veratramine upon treatment with water prior to extraction. Temperature and pH studies, as discussed herein, further support this hypothesis.

The following examples are included for purposes of illustration of certain aspects and embodiments of the present invention.

### A. Harvesting the biomass

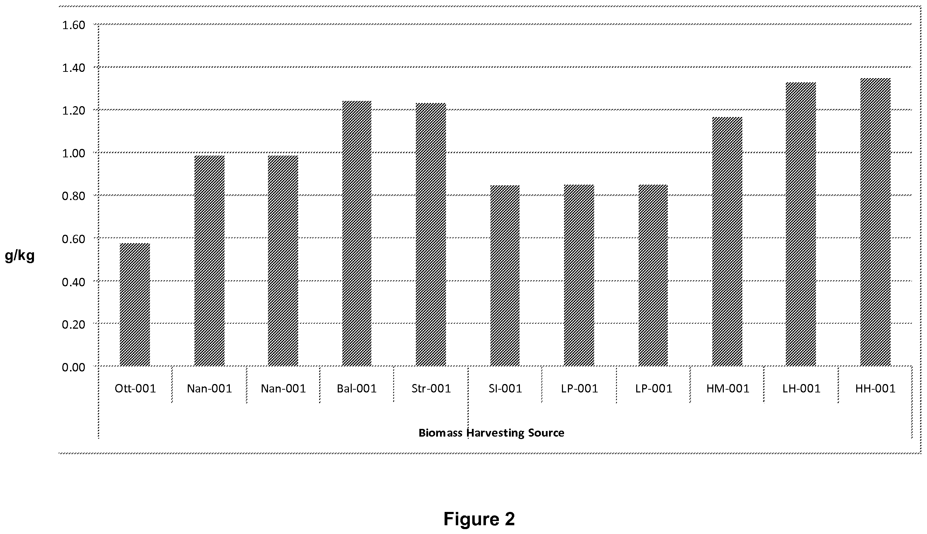

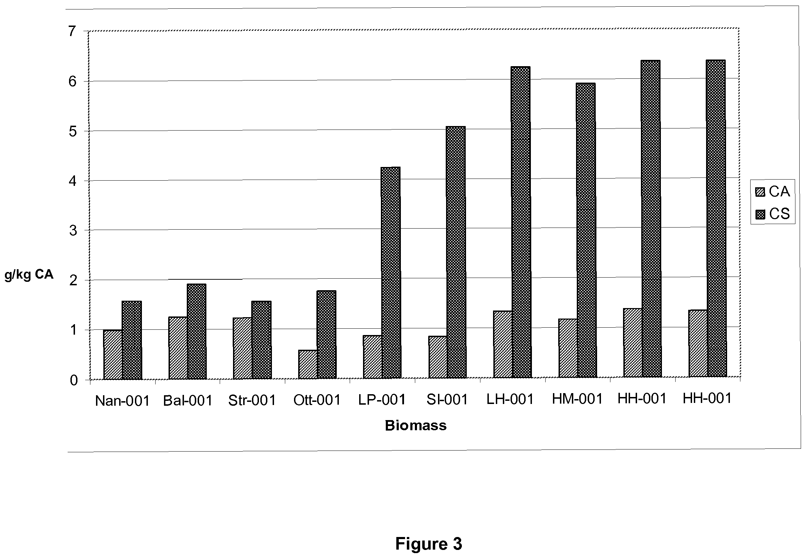

*V. californicum* is typically harvested in August-October of each year. The plant material is dug from the ground manually and/or using farm equipment when and where accessible. Typically, the root bulb, corn, and rhizome are separated from the plant and are chopped manually into small pieces, and further dried over a 2 to 4 week period. The dried material is then milled into fine particles (*i.e.,* the biomass). Moisture content of the fine particles ranges between 7% to 10%. Lots of crude biomass contain varying ampounts of cyclopamine and cycloposine (see Figures 2 and 3). The biomass is stored at approximately 2 °C to 8°C in sealed opaque bags lined with polypropylene prior to extraction.

### B. Analytical Methods

General HPLC Method: Waters Symmetry C18 column, 4.6 × 150 mm, 5 micron, (P/N WAT045905); Mobile Phase: 20/80 MeCN/0.1% TFA at time 0 to 35/65 MeCN/0.1% TFA at 12 min followed by 5 minutes of re-equilibration; Flow rate: 1.5 mL/min; Injection: 10 microliters; Detector: 215 nm; Temperature 40°C.

### C. Exogenous Enzymatic Studies

All enzymes were supplied as either purified isolates or crude extracts and supplied in solution or as solids which were diluted in buffer solutions. The enzyme activity concentrations were calculated based upon information provided by the suppliers on packing information. The appropriate dilutions were made to achieve the desired enzyme contentrations.

The reactions were typically carried out in Eppendorf tubes incubated in temperature controlled heat blocks in a buffered solution at about 37 °C for approximately 22 hours. Exemplary buffers are provided in Table 1.

**Table 1.**

| **Common Name** | **Buffer Range** | **Full Compound Name** |
|---|---|---|
| TAPS | 7.7-9.1 | 3- {[tris(hydroxymethyl)methyl] amino }propanesulfonic acid |
| Bicine | 7.6-9.0 | N,N-bis(2-hydroxyethyl)glycine |
| Tris | 7.5-9.0 | tris(hydroxymethyl)methylamine |
| Tricine | 7.4-8.8 | N-tris(hydroxymethyl)methylglycine |
| HEPES | 6.8-8.2 | 4-2-hydroxyethyl-1-piperazineethanesulfonic acid |
| TES | 6.8-8.2 | 2-{[tris(hydroxymethyl)methyl]amino}ethanesulfonic acid |
| MOPS | 6.5-7.9 | 3-(N-morpholino)propanesulfonic acid |
| PIPES | 6.1-7.5 | piperazine-N,N'-bis(2-ethanesulfonic acid) |
| Cacodylate | 5.0-7.4 | dimethylarsinic acid |
| MES | 5.5-6.7 | 2-(N-morpholino)ethanesulfonic acid |
| Carbonic acid | 7.35-7.45 | Carbonic acid-bicarbonate solution |
| PBS | 7.4 | Phosphate buffer saline |
| Acetate | 3.7-5.6 | Acetic acid-sodium acetate solution |

Pure cycloposine (~7 mg) or a crude *Veratrum* extract containing cycloposine (~16 mg) was dissolved in 500 uL of 10% DMSO in ethanol. 50 uL of the solution containing cycloposine (1 equivalent) and 50 uL of a solution of enzyme (approximately 2 activity unit equivalents) were added to a 450 uL buffer solution (0.1M Tris buffer, pH 7.2; or 0.1 M acetate buffer, pH 5.1). The mixture was heated at 37 °C for 22 hours. At that time, 50 uL of the cloudy reaction suspension was combined with 50 uL MeOH. Enzymatic conversion was determined by HPLC. Results for various exemplary enzymes are summarized in Table 2.

**Table 2.**

| **Enzyme** | **Host Organism** | **Source** | **Conversion of CS to CA** |
|---|---|---|---|
| β-Glucuronidase | Helix pomatia | Calbiochem 347420 | yes |
| β-Glucuronidase | Helix pomatia Type H-1 | Sigma G0715 | yes |
| β-Glucuronidase | Helix pomatia Type HP-2 | Sigma G7017 | yes |
| β-Glucuronidase | Helix pomatia Type H3 | Sigma G8885 | yes |
| β-Glucuronidase | Helix pomatia Type H-5 | Sigma G1512 | yes |
| β-Glucuronidase | Helix aspersa | Sigma G4259 | yes |
| β-Glucuronidase | Patella vulgata Type L-II (keyhole limpet) | Sigma G8132 | yes |
| β-Glucuronidase | Bovine Type 10 from liver | Sigma G0501 | yes |
| β-Glucosidase | Almonds | Sigma G4511 | yes |
| α-Glucosidase | Saccharomyces cerivisiae | Sigma G0660 | not observed |
| β-Glucuronidase | E. coli | Sigma G8162 | not observed |
| β-Glucuronidase | E.coli expressed in E.coli. | Sigma G8295 | not observed |
| α-Glucosidase | Bacillus stearothermophilus | Sigma G3651 | not observed |

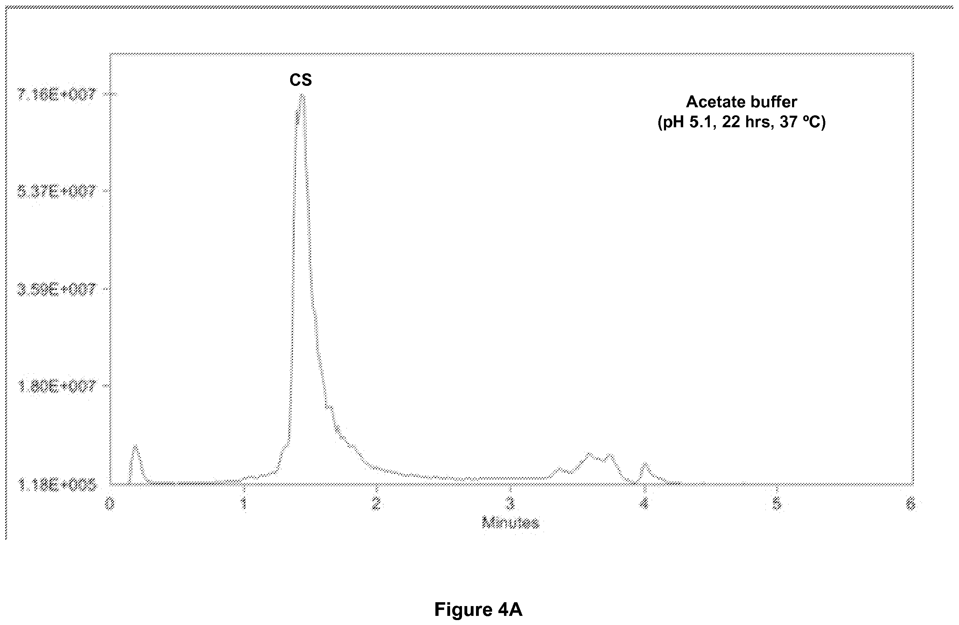

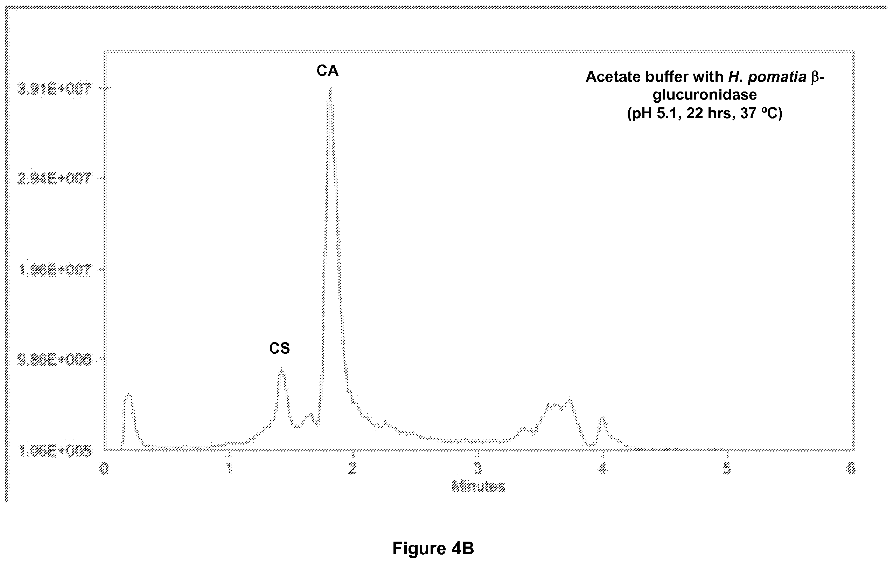

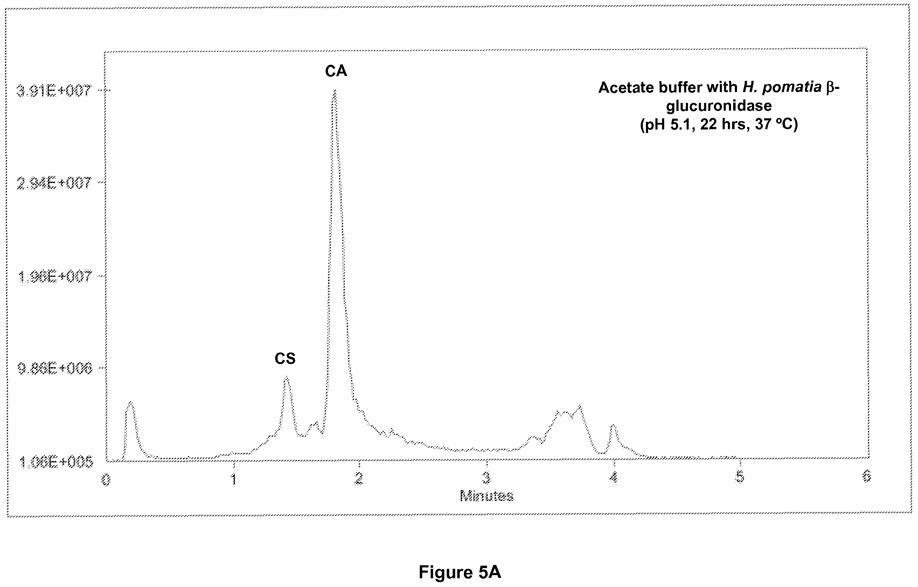

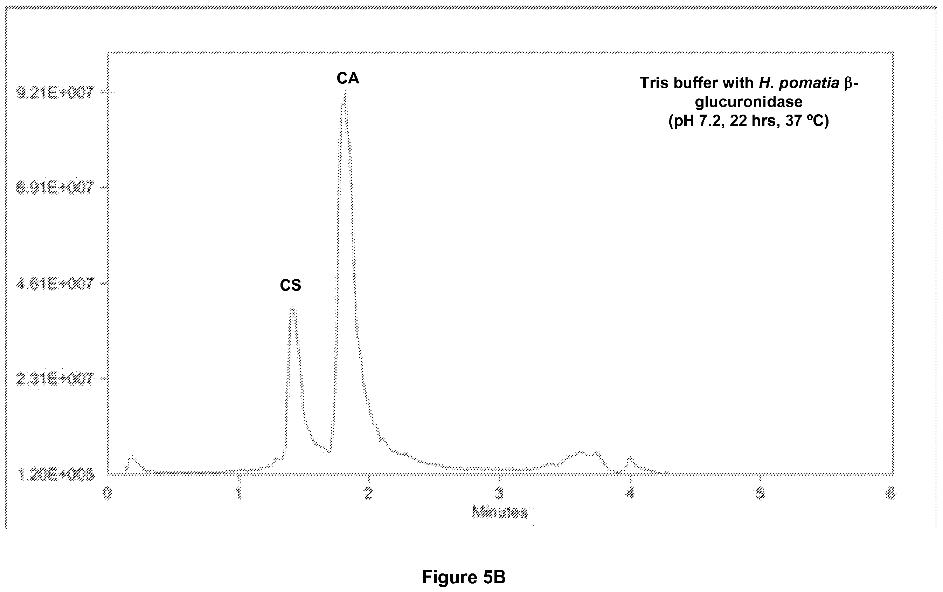

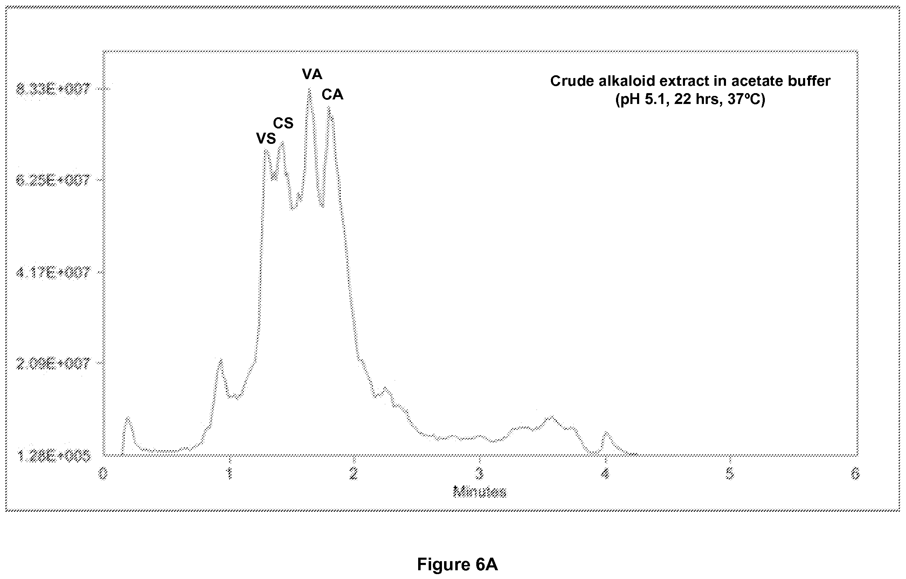

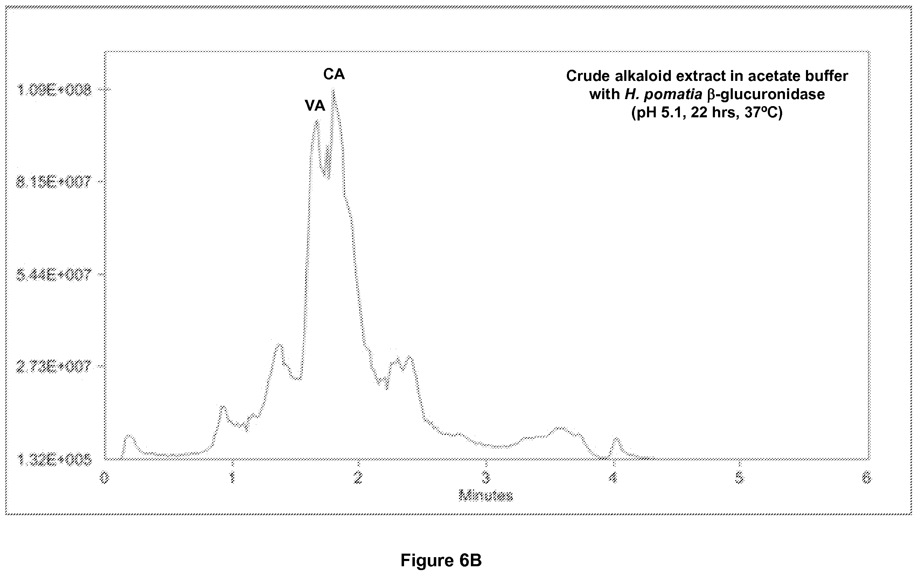

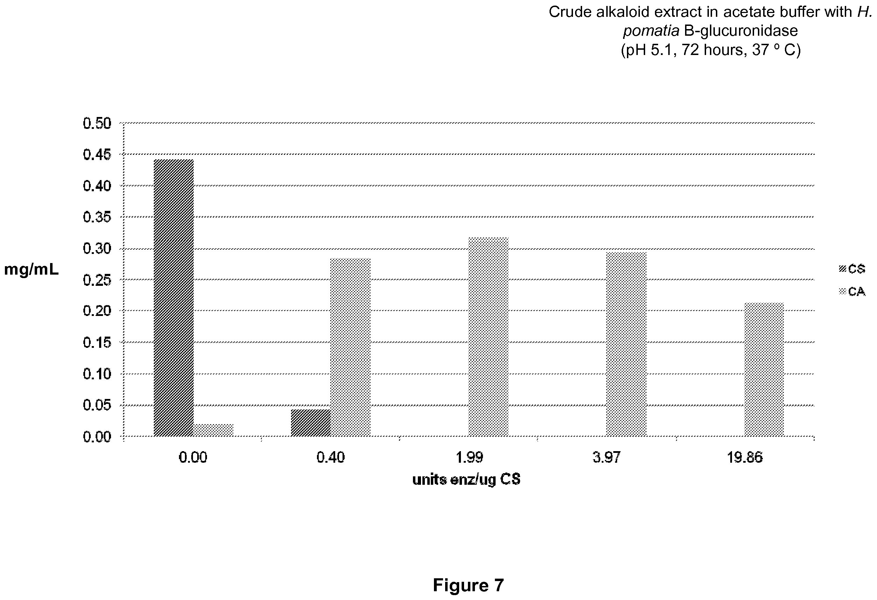

As shown in Figures 4 and 5, purified cycloposine is converted to cyclopamine using *H. pomatia* β-glucuronidase in acetate or Tris buffer. Figures 6 and 7 show similar results for a crude *Veratrum* extract.

In general, it was found that the exogenous enzymes used in this study were effective in converting cycloposine to cyclopamine at about pH 7.2 and about pH 5.1 at about 37 °C, with a pH of about 5.1 affording a more complete conversion (see Figure 5). It was also observed that the eukaryotic-derived enzymes used in this study were more effective than the prokaryote-derived enzymes. In reactions using crude alkaloid extract, conversion of veratrosine to veratramine was also observed (see Figure 6).

### D. First Protocol

### (i) Extraction of the biomass

Extractions using an organic solvent with the addition of 5% aqueous ammonium hydroxide provided better yields of cyclopamine versus using the organic solvent alone, or using 10% or 20% aqueous ammonium hydroxide with an organic solvent. A 3:1 ratio of organic solvent to 5% aqueous ammonium hydroxide provides a mixture with the biomass that is most uniform and readily mixed. Two extractions generally result in the majority of the cyclopamine being extracted from the biomass; subsequent extractions typically provided more crude extract with less cyclopamine. Cycloposine is also typically present in the extract. The results of various extraction solvents are summarized in Table 3.

**Table 3.**

| Extraction solvent(s) | Cyclopamine (mg/kg) | | | | Cycloposine (mg/kg) estimated from CA standard curve | | | | CA + CS (mg/kg) |
|---|---|---|---|---|---|---|---|---|---|
| | 1st | 2nd | 3rd | total | 1st | 2nd | 3rd | tota 1 | |
| 5% AcOH | 625 | 209 | 22 | 855 | 226.3 | 65.6 | 0.0 | 292 | 1147 |
| EtOH | 495 | 15 | 0 | 510 | 195.5 | 0.0 | 0.0 | 196 | 706 |
| IPA | 292 | 25 | 0 | 317 | 71.8 | 0.0 | 0.0 | 72 | 389 |
| acetonitrile | 48 | 0 | 0 | 48 | 0.0 | 0.0 | 0.0 | 0 | 48 |
| pyridine | 469 | 85 | 0 | 554 | 199.4 | 10.9 | 0.0 | 210 | 765 |
| THF | 425 | 7 | 0 | 431 | 112.6 | 0.0 | 0.0 | 113 | 544 |
| dioxane | 226 | 0 | 0 | 226 | 81.0 | 0.0 | 0.0 | 81 | 307 |
| MEK | 188 | 0 | 0 | 188 | 0.0 | 0.0 | 0.0 | 0 | 188 |
| DME | 138 | 263 | 0 | 401 | 2.9 | 0.0 | 0.0 | 3 | 404 |
| DCM/5%NH₄OH | 651 | 235 | 44 | 930 | 270 | 288 | 60 | 618 | 1548 |
| MTBE/5%NH₄OH | 681 | 292 | 51 | 102 4 | 235 | 235 | 85 | 555 | 1579 |
| benzene/5%NH₄OH | 790 | 282 | 4 | 107 6 | 0 | 0 | 0 | 0 | 1076 |
| toluene/5%NH₄OH | 770 | 213 | 0 | 983 | 0 | 0 | 0 | 0 | 983 |
| cyclohexane/5%NH₄OH | 770 | 354 | 47 | 117 1 | 0 | 0 | 0 | 0 | 1171 |

| | Cyclopamine (mg/kg) | | | | Cycloposine (mg/kg) estimated from CA standard curve | | | | |
|---|---|---|---|---|---|---|---|---|---|
| MIBK/5% NH₄OH | 608 | 347 | 26 | 981 | 283 | 283 | 83 | 649 | 1630 |
| EtOAc/5%NH₄OH | 345 | 191 | 17 | 553 | 204 | 211 | 9 | 424 | 977 |
| iPrOAc/5%NH₄OH | 365 | 223 | 15 | 603 | 147 | 236 | 122 | 505 | 1108 |
| 2-MeTHF/5%NH₄OH | 373 | 303 | 31 | 707 | 343 | 303 | 58 | 704 | 1411 |
| MTBE | 104 | 6 | nd | 110 | 0 | 0 | nd | 0 | 110 |
| MTBE/5%NH₄OH | 681 | 292 | 51 | 102 4 | 235 | 235 | 85 | 555 | 1579 |
| MTBE/5%Na₂CO₃ | 395 | 286 | 58 | 739 | 116 | 172 | 93 | 381 | 1120 |
| MTBE/5%AcOH | 191 | 52 | 52 | 295 | 0 | 0 | 0 | 0 | 295 |
| cyclohexane | 6 | 0 | nd | 6 | 0 | 0 | nd | 0 | 6 |
| cyclohexane/5%NH₄OH | 770 | 354 | 47 | 117 1 | 0 | 0 | 0 | 0 | 1171 |
| cyclohexane/5%Na₂CO₃ | 443 | 325 | 82 | 850 | 0 | nd | nd | 0 | 850 |
| cyclohexane/5%AcOH | 0 | 0 | 0 | 0 | 0 | 0 | 0 | 0 | 0 |

Large Scale Extraction: Approximately 6 kg of biomass was suspended in a mixture of dichloromethane (CH₂Cl₂) containing 5% aqueous ammonium hydroxide at room temperature for 16 hours. The solvent was decanted and concentrated to a paste, which was taken up in a mixture of THF/hexane (1:3). After standing for 2 to 3 days, the solvent was decanted and concentrated to a viscous oil. The crude oil was loaded onto a silica gel column and eluted with EtOAc/CH₂Cl₂/MeOH/Et₃N (8:1:1:0.1). The enriched fraction with cyclopamine was concentrated to ¼ of the volume and the precipitated cyclopamine was filtered. More cyclopamine was obtained by further precipitation with acetone. The combined cyclopamine was recrystallized in hot MeOH. This protocol typically delivers 1 g of cyclopamine per kg of dry biomass. The purity of cyclopamine obtained by this process exceeds 95%, as determined by HPLC.

### (ii) Purification of the extract

11 g of the crude extract (obtained as described above) was stirred overnight in 50 mL of methanol, followed by filtration to obtain a crude filtrate. Celite (25 g) was added to the filtrate and the mixture was concentrated to dryness by rotary evaporation. The resulting dry powder was eluted with ethyl acetate to obtain a first crude fraction containing a mixture of veratramine and cyclopamine, followed by elution with methanol to obtain a second crude fraction containing cycloposine. The second fraction was concentrated to 4 grams of a crude material.

The crude material obtained from the second fraction was loaded to a silica gel column and eluted with 1:2:4 MeOH:EtOAc:Hexanes (0.5% triethylamine as an additive) to obtain cycloposine (85% pure as determined by HPLC). The crude cycloposine was triturated with acetone and decanted, and further purified by silica gel chromatography (1:2:4 MeOH:EtOAc:Hexanes elutant, containing 0.5% triethylamine as an additive) to obtain purified cycloposine, which was recrystallized from methanol to obtain 200 mg of cycloposine, 97% pure as determined by HPLC.

### B. Second Protocol - Aqueous Pre-treatment

It was discovered that treatment of the *Veratrum* plant material with an aqueous solution prior to extraction decreases the amount of cycloposine and veratrosine present in the plant material and increases the yield of isolated cyclopamine and veratramine. This protocol represents a 2 to 5 fold increase in the yield of cyclopamine compared to the above-described protocol.

### (i) Extraction with 100% Methanol

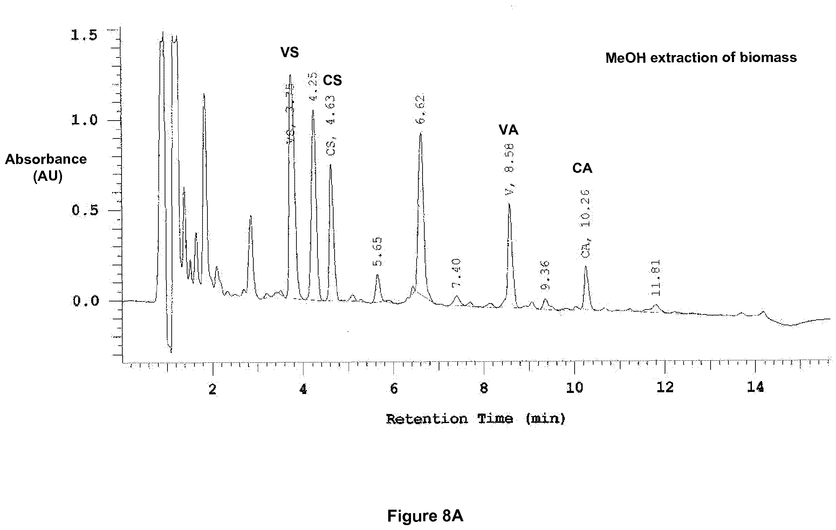

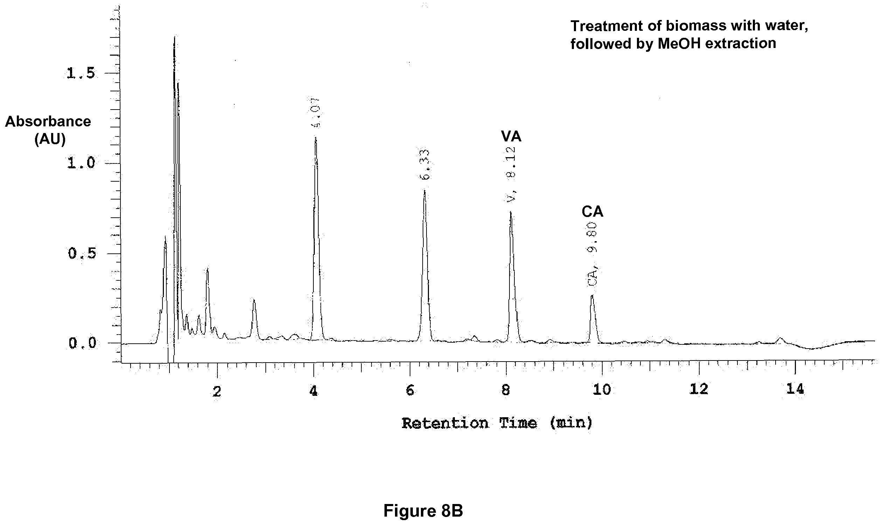

13.2 g of dried biomass was exhaustively extracted with methanol (8-10 volumes of MeOH mixed per weight with milled/dried *Veratrum Californicum,* and the mixture shaken at room temperature for 24 hours) and filtered. The extraction process was repeated two additional times on the filtered biomass, for a total of three extractions. HPLC analysis showed 22 mg cyclopamine (CA) (0.17% of the total weight of the biomass) and 65 mg (0.49%) cycloposine (CS) (Figure 8). The theoretical total if all cycloposine is converted to cyclopamine is 0.66% (the theoretical total is determined by taking the sum of CA content and CS content corrected for molecular weight differences (e.g., CA + CS^{∗}(mwCA/mwCS) = theoretical CA). The percentage of extractable solids was 25.7%.

### (ii) Treatment with Water, followed by 100% Methanol Extraction

10.0 g of dried biomass was stirred at room temperature with 50 mL of water for about 1.5 hours. It was observed that cycloposine (CS) in the aqueous filtrate disappeared in less than an hour, as monitored by HPLC. 50 mL of MeOH was then added and the slurry was filtered giving 78 mL of filtrate containing 313 mg of cyclopamine (47% of the theoretical maximum of 666 mg). The biomass was then re-extracted with 65 mL of MeOH giving another 18.2 mg of cyclopamine (CA), for a total of 331.2 mg.

Figure 8 shows HPLC traces of the extracts obtained from (a) 100% MeOH and (b) from treatment with water followed by extraction with 100% MeOH. The chromatograms indicate that cycloposine (CS) and veratrosine (VS) were reduced and both cyclopamine (CA) and veratramine (V) increased in the sample treated with water.

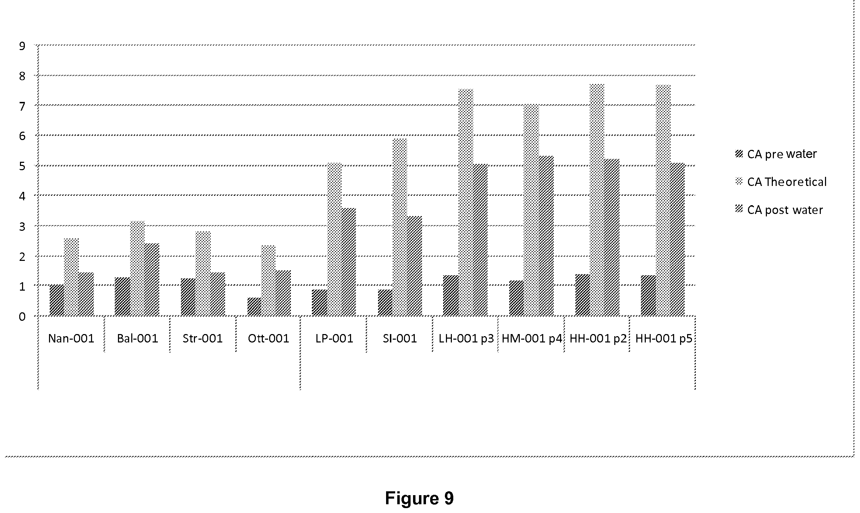

Figure 9 depicts the conversion of cycloposine to cyclopamine from various biomass samples. In each case, upon treatment with water followed by MeOH extraction, the overall concentration of cyclopamine present in the biomass samples increased .

### (iii) Extraction with 50% Methanol in Water

Two 100 g lots of biomass were stirred at room temperature overnight, one with 500 mL of MeOH and the other with 500 mL of 1:1 MeOH/water. The slurries were filtered and each lot of biomass re-extracted with 300 mL of the same solvent. The MeOH extraction returned 152 mg cyclopamine while the MeOH/water extract contained 349 mg of cyclopamine.

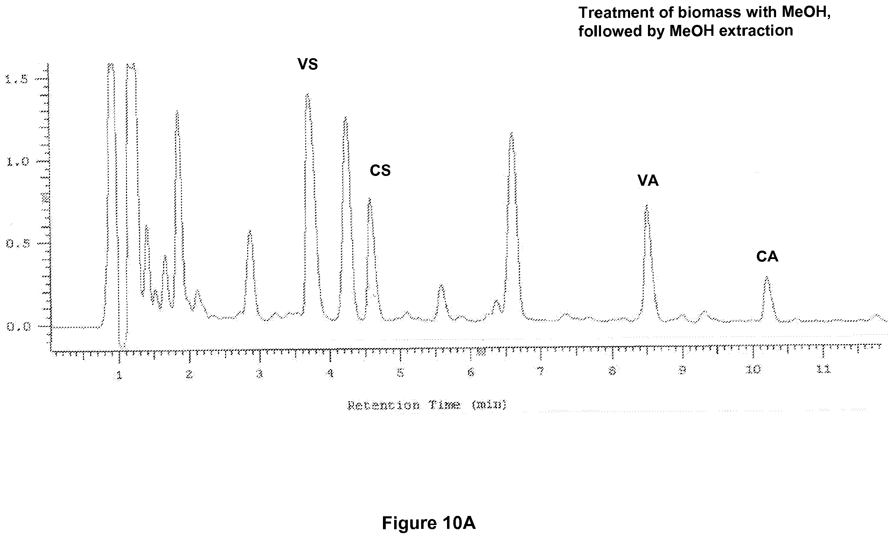

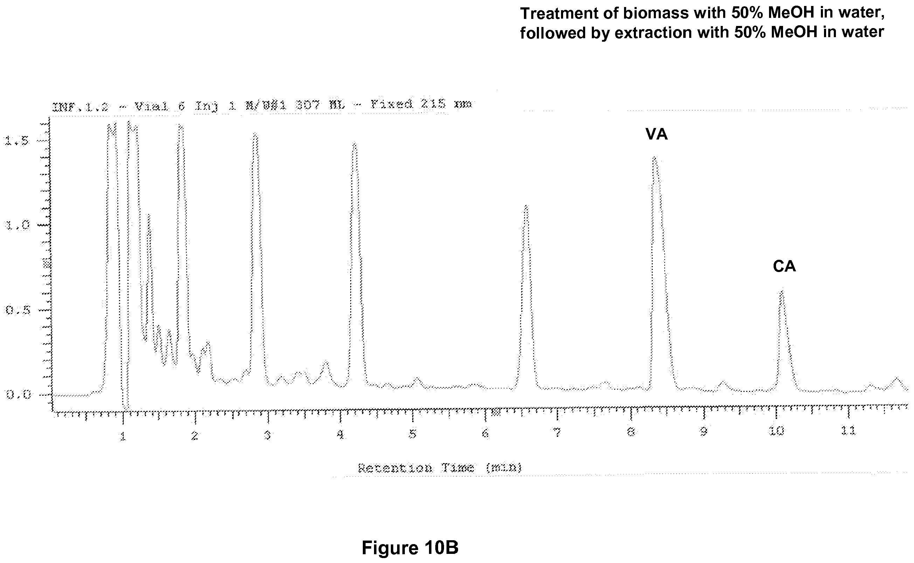

Figure 10 shows HPLC traces of the extracts obtained from (a) 100% MeOH and (b) from treatment with 50% MeOH in water, followed by extraction with 50% MeOH in water. The chromatograms indicate that cycloposine (CS) and veratrosine (VS) were reduced and both cyclopamine (CA) and veratramine (VA) increased in the sample treated with 50% methanol in water.

### (iv) Effect of Temperature during Treatment with an Aqueous Solution

Two samples of biomass (2.0 g of biomass containing 0.17% CA and 0.49% CS, CS/CA ratio = 2.88, as determined by HPLC analysis of a MeOH extract) were each placed in 50 mL centrifuge tubes along with 10 mL of 50% MeOH in water. The tubes were shaken and one tube was placed in a 40°C bath and the other tube left at room temperature. 1 mL samples were removed and filtered into HPLC vials at various times. The concentration of cyclopamine (CA) and cycloposine (CS) in each sample was determined by HPLC (see Table 4).

**Table 4.**

| **Time (Min)** | **Temp °C** | **mg/mL CA** | **mg/mL CS** | **CS/CA Ratio** |
|---|---|---|---|---|
| 0 | --- | --- | --- | 2.88 |
| 45 | 22 | 0.47 | 0.17 | 0.36 |
| 45 | 40 | 0.06 | 0.03 | 0.47 |
| 165 | 22 | 0.69 | 0.08 | 0.11 |
| 165 | 40 | 0.26 | 0.05 | 0.20 |
| 270 | 22 | 0.67 | 0.0 | 0.0 |
| 270 | 40 | 0.55 | 0.07 | 0.14 |

In a separate experiment, the dried biomass was heated to 100 °C, then cooled to room temperature and treated with water. HPLC analysis of a MeOH extract showed no appreciable conversion of cycloposine to cyclopamine (i.e., the CS/CA ratio was unchanged). The results indicate that conversion of cycloposine to cyclopamine in the biomass is halted or diminished if the biomass is first heated to 100 °C prior to treatment with water.

### (v) Effect of pH during Treatment with an Aqueous Solution

Two samples of biomass (2.0 g of biomass containing 0.17% CA and 0.49% CS, CS/CA ratio = 2.88, as determined by HPLC analysis of MeOH extract) were each placed in 50 mL centrifuge tubes along with 5 mL of MeOH and 5 mL of 0.5 M sodium carbonate solution. The tubes were shaken and one tube was placed in a 40°C bath and the other tube left at room temperature. 1 mL samples were removed and filtered into HPLC vials at various times. After 45 minutes, HPLC analysis of both samples showed no appreciable conversion of cycloposine to cyclopamine (i.e., the CS/CA ratio was unchanged). The results indicate that conversion of cycloposine to cyclopamine in the biomass is halted or diminished if treated with base or if the aqueous solution contacting the biomass is a basic solution.

### (vi) Treatment with Water followed by Extraction with basic or neutral Methanol: Experiments using Sodium Carbonate

Two 100 g samples of biomass were weighed into each of two 750 mL bottles. 500 mL of water was added to each and the samples were occasionally shaken over a 1.5 hour time period. The two samples were then filtered separately giving 300 mL of aqueous filtrate with 14.5 g of solids and only a trace amount of cyclopamine (CA). Cycloposine (CS) was not observed in the filtrate (as determined by HPLC) suggesting that it had all been converted to cyclopamine.

The first sample (designated "neutral") was extracted three times with methanol (3 × 150 mL) at 40 °C. The combined neutral extracts contained 0.50 g of cyclopamine (75% of the theoretical maximum of 0.66 g). A fourth extraction of the biomass with 300 mL of boiling MeOH brought the total to 0.54 g (82% of theoretical).

The second sample (designated "basic") was basified with 25 mL of a 1 M sodium carbonate solution. This sample was then extracted three times with methanol (3 × 150 mL) at 40 °C. An additional 25 mL of a 1 M sodium carbonate solution was added to the sample prior to the second basic extraction but not prior to the third extraction. The combined basic extracts contained 0.62 g of cyclopamine (94% of the theoretical maximum of 0.66 g). A fourth extraction of the biomass with 300 mL of boiling MeOH brought the total to 0.67 g (100% of theoretical).

*Large scale study:* 1000 g of biomass and 5.0 L of deionized water were added to a 10 L round bottom flask and the mixture was slowly mixed at room temperature for 1.5 hr. This slurry was filtered on #417 hardened paper and through a Celite 545 bed. Analysis of the aqueous filtrate indicated 0.069 g of cyclopamine present (1% of total).

The moist biomass was returned to the round bottom flask and treated with 125 mL of 2 M sodium carbonate and 4 L of MeOH. The mixture was slowly mixed in a 40 °C bath for 1.5 hour and then filtered using a 24 cm Buchner funnel with #417 paper. Filtration in this case was very rapid. The extraction procedure was repeated two additional times with MeOH. 250 mEq of sodium carbonate was added to the sample prior to the second extraction but not prior to the third. The results are summarized in Table 5 below.

**Table 5.**

| **Extraction no.** | **Volume (L)** | **[cyclopamine]^{a}; g/L** | **Cyclopamine (g)^{b} calculated** | **Cyclopamine (g)^{c} actual** |
|---|---|---|---|---|
| 1 | 4.0 | 0.99 | 3.96 | 3.96 |
| 2 | 5.1 | 0.45 | 2.29 | 2.29 |
| 3 | 3.8 | 0.11 | 0.41 | 0.41 |
| Total | 12.9 | 1.55 | 6.66 | 6.66 |

| | | | | |
|---|---|---|---|---|
| ^{a} as observed by HPLC; theoretical/calculated amount; ^{c} amount obtained upon extraction | | | | |

The combined extracts were found to contain 151 g of solids and 100% of the theoretical maximum of cyclopamine available in the biomass sample. Cyclopamine in the extract had a purity of 4.4% (as determined by HPLC).

### (vii) Treatment with Water followed by Extraction with basic or neutral Methanol: Experiments using Triethylamine

Two 15 g samples of biomass were placed in each of two 250 mL centrifuge bottles and treated with 150 mL water with occasional shaking for 1 hour. After this time the two samples were centrifuged and the water decanted. 100 mL of water was recovered and 2.6 mg of cyclopamine was found in the aqueous extracts. The conversion of cycloposine to cyclopamine appeared to be complete since there was only trace amounts of cycloposine in the aqueous extract.

One sample (designated "neutral") was extracted four times with 50 mL of MeOH. Each extraction entailed shaking the sample with the solvent, centrifuging, and vacuum filtering the supernatant through #1 filter paper. Any biomass retained on the paper was returned to the bottle and the process repeated. The combined neutral MeOH extracts contained 61 mg of cyclopamine (61% of the theoretical).

The second sample (designated "basic") was treated as above except that the extraction solvent was a 95:5 mixture of MeOH and triethylamine. Filtration of the basic sample was slower than the neutral sample. However, the concentration of cyclopamine in the basic extracts was higher. The combined basic MeOH extracts contained 73 mg of cyclopamine (73% of theoretical).

### (viii) Optimizing the Extraction Step

### (a) Liquid-liquid Extraction with EtOAc/Hexanes/NaOH

A extract containing approximately 248 mg of cyclopamine in 50% MeOH was evaporated under reduced pressure to remove the majority of the methanol. The pH of the solution was adjusted to 9.2 with the addition of NaOH and the mixture was extracted with 50% ethyl acetate in hexanes (200 mL). An emulsion formed in the organic phase, and the combined organic and emulsion layers were passed through anhydrous sodium sulfate in a filter into a clean round bottom flask. Evaporation of the extract left a pale yellow solid (0.5 g) containing 154 mg of cyclopamine (29% pure, 62% recovery). HPLC analysis of the aqueous layer indicated no cyclopamine was present.

### (b) Liquid-liquid Extraction with EtOAc/Hexanes/Sodium Carbonate

555 mL of methanol/water extracts containing approximately 169 mg of cyclopamine was concentrated under reduced pressure to yield 275 mL of slurry. The pH of the slurry was adjusted to 9.5 with the addition of 10 mL of a 1M sodium carbonate solution. The slurry was then extracted with 50% ethyl acetate in hexanes (2 × 150 mL) and the emulsion layer was separated from the organic layer with each extraction. The combined emulsion layers from the two extractions were centrifuged giving a well-defined organic layer which was combined with the rest of the organic layer in a round bottom flask. No desiccant drying was performed. Evaporation provided 0.88 g of yellow solids containing 166 mg of cyclopamine (18.8% pure; 98% recovery). HPLC analysis of the aqueous layer indicated no cyclopamine was present.

### (c) Liquid-liquid Extraction with a 1 kg extract

12.9 L of a MeOH extract was concentrated under reduced pressure in a 10 L rotoevaporatory flask using a 55-60 °C bath. 1.55 L of black suspension was decanted from residual black tar into a separatory funnel and extracted with 1 × 1000 mL and 3 × 500 mL of 1:1 EtOAc/hexanes (the tar was only sparingly soluble in EtOAc at room temperature). By the fourth extraction, only 32 mg of cyclopamine was present in the aqueous phase (corresponding to 0.5% of the total). A total of 1.8 L of the extract was obtained and HPLC analysis indicated 3.9 g of cyclopamine was present in the extract having 14.3 % purity (59% of theoretical). This result suggested that the remaining cyclopamine (2.7 g) must be present in the black tar. Therefore, the black tar remaining in the flask plus additional tar left after decanting the liquid from the aqueous layer was dissolved in 725 mL of MeOH and analyzed by HPLC. Cyclopamine was present in the tar in 2.78 g in 20 g of solids, corresponding to 14% purity.

*Reextraction of the black tar with EtOAc*/*hexanes:* 50 mL of the methanolic tar solution was diluted to 250 mL with water and re-extracted with 1:1 EtOAc/hexanes. Sodium sulfate was added to improve the separation of the water/organic layers. A single extraction with 100 mL of solvent yielded 70% of the cyclopamine in the organic phase. In a second experiment, 100 mL of methanolic tar solution was evaporated to 50 mL and diluted with 400 mL of water. This was extracted 2 × 100 mL with 1:1 EtOAc/hexanes leaving only 2% of the cyclopamine in the aqueous phase and 72% in the organic phase. The remaining 26% was left in the tar.

*Drying the tar on silica gel:* 2 g of the methanolic tar solution containing 7.6 mg of cyclopamine was dried on 2 g of silica gel. 0.5 g of the mixture was placed in an empty 3 mL solid phase extraction cartridge and extracted with 5 mL of EtOAc. A majority of the cyclopamine remained on the column. A second experiment was conducted using EtOAc/triethylamine (TEA) as an elutant, with approximately 80% of the cyclopamine eluting from the silica. A third experiment was conducted using 35% acetone with 0.5% TEA, with approximately 84% cyclopamine eluting from the silica.

*Drying the tar on Celite:* 2 g of the methanolic tar solution containing 7.6 mg of cyclopamine was dried on 2 g of Celite. Elution with 35% EtOAc with TEA gave a product with about 49% purity, with 13% of the cyclopamine remaining on the Celite. Elution with 100% EtOAc, 50% acetone in hexanes and 100% acetone each gave nearly quantitative recovery of cyclopamine from the Celite, providing cyclopamine having 28%, 22% and 20% purity respectively.

The remaining methanolic tar solution (525 mL, 2.0 g of cyclopamine in 14.2 g of solids) was evaporated to approximately 300 mL and mixed with 180 g of Celite 503 in a 1 L lyophilization jar. An additional 100 mL of MeOH was removed under reduced pressure and then the mixture was placed on a large drying table and dried in a 100 C oven to constant weight. Analysis showed that cyclopamine content was 1.01% in the bulk powder (194 g).

17 g of the bulk powder was extracted with 130 mL of EtOAc in a 60 mL glass-sintered funnel. The resulting amber extract contained 158 mg (93% recovery) of the cyclopamine in 620 mg of solids (25% purity). A MeOH wash of the Celite powder provided 13 mg of cyclopamine in 586 mg of solids.

### (ix) Purification of cyclopamine-containing extracts

### (a) Step 1: Silica Gel Chromatography

Thin layer chromatography (TLC) experiments indicated good separation of cyclopamine and veratramine using 50% acetone in hexanes with 5% triethylamine (TEA) as elutant (Rf of cyclopamine = 0.4).

Separation by silica gel chromatography was performed using 40% acetone in hexanes with 5% triethylamine on a 1.2 × 15 cm silica Biotage 12 M column. The column was washed with acetone and equilibrated with hexanes. The first liquid-liquid extraction product was dissolved in 10 mL of ethyl acetate and loaded on the column. 20 mL fractions were collected. Most of the veratramine eluted in fraction 2, while cyclopamine eluted in fractions 3 and 4. Cyclopamine in the sample of combined fractions 3 and 4 had an assay purity of 73.1% as determined by HPLC (normalized recovery = 99%; recovery based on the 145 mg of cyclopamine theoretically present in the starting material = 60%).

A second small-scale silica gel chromatography experiment was conducted using 35% acetone in hexanes with 5% triethylamine mobile phase on a 1.2 × 15 cm silica Biotage 12 M column. The column was equilibrated with hexanes and 0.80 g of liquid-liquid extraction product containing 150 mg of cyclopamine was dissolved in 5 mL of EtOAc and loaded onto the column. The mobile phase was run at 5 mL/min and 20 mL fractions collected. Veratramine and cyclopamine were well separated in the experiment and only 5 mg of cyclopamine was lost in the early fractions containing veratramine. The cyclopamine pool contained 141 mg of cyclopamine (94% recovery) at 76% purity with only 0.5 area percent veratramine. Normalized recovery was 97%.

### (b) Step 2: Trituration

Partial evaporation of the cyclopamine pool (provided from the chromatography experiment conducted using 35% acetone in hexanes with 0.5% triethylamine mobile phase) caused cyclopamine to precipitate out.

The pool was evaporated to dryness and then sonicated for 1 minute with 10 mL of 35% acetone in hexanes. The insoluble product was recovered on a glass sintered filter and dried, providing a white solid (110 mg) with 95% assay purity and 93.8% chromatographic purity. 22.6 mg of cyclopamine was present in the filtrate (84% recovery).

### (c) Step 3: Crystallization

Small scale experiments on the trituration product indicated that cyclopamine crystallizes from hot ethanol, isopropanol and toluene and can be forced to recrystallize from THF at room temperature by the addition of hexanes. Both ethanol and toluene can also be used to remove water of hydration by azeotropic removal of water to provide anhydrous cyclopamine.

100 mg of the trituration product was dissolved in 5 mL of hot toluene. Boiling reduced the volume to approximately 3 mL and then the solution was chilled overnight at 4 °C. Crystals were collected on a small glass-sintered filter and washed with a minimal amount of cold toluene and 5 mL of room temperature hexanes. The crystals had an assay purity of 97.7% and a chromatographic purity of 96.4%.

### E. Manufacturing protocol

Approximately 200 kg biomass was added to a 1000 L reaction vessel/extraction tank and approximately 45 L water per 18 kg bag of biomass was added. The mixture was held at room temperature without agitation for 2.5 hours (starting at the time that loading was complete). At this time, the water was removed from the vessel by filtration.

8 liters of MeOH per kg of biomass was added to the vessel containing the biomass and the mixture was maintained at 40-50 °C with agitation for a minimum of 4 hours, and the MeOH extract collected. This extraction process was repeated 2 times to achieve exhaustive yields.

The methanol extracts were concentrated until solids began to precipitate. The solids were redissolved in a minimum volume of MeOH and filtered though a small plug of Celite to remove any insoluble materials. The resulting MeOH solution was mixed with Celite and dried to a powder. An enriched cyclopamine containing extract was obtained by exhaustive elution of the powder with EtOAc heated to 40-50 °C, and the EtOAc eluent was concentrated to a crude material.

The crude material was purified by silica gel chromatography using 35% acetone in heptanes (containing 0.5% triethylamine as an additive) as eluent. Pooled fractions from this purification step provided cyclopamine in 70-80% purity as determined HPLC. The purity of the cyclopamine material was increased to an excess of 95% by trituration with EtOAc.

## Claims

1. A method of isolating a deglycosylated *Veratrum* alkaloid from *Veratrum* plant material, comprising the steps of:
(i) providing a *Veratrum* plant material comprising a glycosylated *Veratrum* alkaloid;
(ii) prior to an extracting step, contacting the *Veratrum* plant material with an aqueous solution at a pH between 5 and 7.5, the aqueous solution comprising greater than 25% water; and
(iii) extracting the *Veratrum* plant material with a solvent to provide an extract comprising the deglycosylated *Veratrum* alkaloid.

2. The method according to claim 1, wherein the aqueous solution is buffered.

3. The method according to claim 1, wherein the aqueous solution is neutral.

4. The method according to claim 1, wherein the aqueous solution is 100% water.

5. The method according to claim 1, wherein the aqueous solution is at a temperature of below about 100 °C.

6. The method according to any preceding claim, wherein the glycosylated *Veratrum* alkaloid is cyloposine and the deglycosylated *Veratrum* alkaloid is cyclopamine, or wherein the glycosylated *Veratrum* alkaloid is veratrosine and the deglycosylated *Veratrum* alkaloid is veratramine.

7. The method according to any preceding claim, wherein the contacting step comprises contacting the *Veratrum* plant material with an aqueous solution for at least about 10 minutes.

8. The method according to any preceding claim, wherein the solvent comprises one or more organic solvents preferably selected from the group consisting of an organic alcohol, an ester, a ketone, an ether, a halogenated hydrocarbon, a hydrocarbon, an aromatic and a heteroaromatic, and a mixture of two or more thereof.

9. The method according to claim 8, wherein the organic solvent is selected from the group consisting of methanol, ethanol, propanol, isopropanol, 2-butanol, n-butanol, acetone, methyl ethyl ketone, ethyl acetate, isopropyl acetate, dichloromethane, dichloroethane, chloroform, anisole, benzene, toluene, xylenes, hexanes, heptanes, pentanes, tetrahydrofuran, dioxane and diethyl ether, preferably methanol.

10. The method according to any preceding claim, wherein the solvent comprises a mixture of one or more organic solvents and water, and/or a base.

11. The method according to claim 10, wherein the solvent comprises a mixture of one or more organic solvents and a base selected from the group comprising: an aqueous solution of ammonium hydroxide, sodium hydroxide, sodium carbonate, sodium bicarbonate, sodium acetate, sodium citrate, potassium hydroxide, potassium carbonate, potassium bicarbonate, potassium sodium tartrate, and lithium hydroxide; or wherein the base is selected from the group comprising: triethylamine, diethylisopropyl amine and pyridine.

12. The method according to claim 11, wherein the organic solvent is methanol.

## Patentansprüche

1. Verfahren zum Isolieren eines deglykosylierten *Veratrum-Alkaloids* aus *Veratrum-*Pflanzenmaterial, umfassend die folgenden Schritte:
(i) Bereitstellen eines *Veratrum*-Pflanzenmaterials, umfassend ein glykosyliertes *Veratrum-*Alkaloid;
(ii) vor einem Extraktionsschritt, Kontaktieren des *Veratrum*-Pflanzenmaterials mit einer wässrigen Lösung bei einem pH zwischen 5 und 7,5, wobei die wässrige Lösung mehr als 25 % Wasser umfasst; und
(iii) Extrahieren des *Veratrum*-Pflanzenmaterials mit einem Lösungsmittel, um einen Extrakt bereitzustellen, der das deglykosylierte *Veratrum-*Alkaloid umfasst.

2. Verfahren nach Anspruch 1, wobei die wässrige Lösung gepuffert ist.

3. Verfahren nach Anspruch 1, wobei die wässrige Lösung neutral ist.

4. Verfahren nach Anspruch 1, wobei die wässrige Lösung 100 % Wasser ist.

5. Verfahren nach Anspruch 1, wobei die wässrige Lösung eine Temperatur von unter etwa 100 °C aufweist.

6. Verfahren nach einem vorhergehenden Anspruch, wobei das glykosylierte *Veratrum-*Alkaloid Cyloposin ist und das deglykosylierte *Veratrum-*Alkaloid Cyclopamin ist, oder wobei das glykosylierte *Veratrum-*Alkaloid Veratrosin ist und das deglykosylierte *Veratrum-*Alkaloid Veratramin ist.

7. Verfahren nach einem vorhergehenden Anspruch, wobei der Kontaktierungsschritt das Kontaktieren des *Veratrum*-Pflanzenmaterials mit einer wässrigen Lösung für zumindest etwa 10 Minuten umfasst.

8. Verfahren nach einem vorhergehenden Anspruch, wobei das Lösungsmittel ein oder mehrere organische Lösungsmittel umfasst, die vorzugsweise ausgewählt sind aus der Gruppe bestehend aus einem organischen Alkohol, einem Ester, einem Keton, einem Ether, einem halogenierten Kohlenwasserstoff, einem Kohlenwasserstoff, einem Aromaten und einem Heteroaromaten und einer Mischung von zwei oder mehreren davon.

9. Verfahren nach Anspruch 8, wobei das organische Lösungsmittel ausgewählt ist aus der Gruppe bestehend aus Methanol, Ethanol, Propanol, Isopropanol, 2-Butanol, n-Butanol, Aceton, Methylethylketon, Ethylacetat, Isopropylacetat, Dichlormethan, Dichlorethan, Chloroform, Anisol, Benzol, Toluol, Xylolen, Hexanen, Heptanen, Pentanen, Tetrahydrofuran, Dioxan und Diethylether, vorzugsweise Methanol.

10. Verfahren nach einem vorhergehenden Anspruch, wobei das Lösungsmittel eine Mischung aus einem oder mehreren organischen Lösungsmitteln und Wasser und/oder einer Base umfasst.

11. Verfahren nach Anspruch 10, wobei das Lösungsmittel eine Mischung aus einem oder mehreren organischen Lösungsmitteln und einer Base umfasst, ausgewählt aus der Gruppe umfassend: eine wässrige Lösung von Ammoniumhydroxid, Natriumhydroxid, Natriumcarbonat, Natriumbicarbonat, Natriumacetat, Natriumcitrat, Kaliumhydroxid, Kalium carbonat, Kaliumbicarbonat, Kaliumnatriumtartrat und Lithiumhydroxid; oder wobei die Base ausgewählt ist aus der Gruppe umfassend: Triethylamin, Diethylisopropylamin und Pyridin.

12. Verfahren nach Anspruch 11, wobei das organische Lösungsmittel Methanol ist.

## Revendications

1. Procédé pour isoler un alcaloïde de *Veratrum* déglycosylé à partir d'un élément végétal du *Veratrum*, comprenant les étapes de :
(i) fourniture d'un élément végétal du *Veratrum* comprenant un alcaloïde de *Veratrum* glycosylé ;
(ii) avant une étape d'extraction, la mise en contact de l'élément végétal de *Veratrum* avec une solution aqueuse à un pH compris entre 5 et 7,5, la solution aqueuse comprenant plus de 25 % d'eau ; et
(iii) l'extraction de l'élément végétal de *Veratrum* avec un solvant pour fournir un extrait comprenant l'alcaloïde de *Veratrum* déglycosylé.

2. Procédé selon la revendication 1, dans lequel la solution aqueuse est tamponnée.

3. Procédé selon la revendication 1, dans lequel la solution aqueuse est neutre.

4. Procédé selon la revendication 1, dans lequel la solution aqueuse est constituée de 100 % d'eau.

5. Procédé selon la revendication 1, dans lequel la solution aqueuse est à une température inférieure à environ 100 °C.

6. Procédé selon l'une quelconque des revendications précédentes, dans lequel l'alcaloïde de *Veratrum* glycosylé est la cyloposine et l'alcaloïde de *Veratrum* déglycosylé est la cyclopamine, ou dans lequel l'alcaloïde de *Veratrum* glycosylé est la vératrosine et l'alcaloïde de *Veratrum* déglycosylé est la vératramine.

7. Procédé selon l'une quelconque des revendications précédentes, dans lequel l'étape de mise en contact comprend la mise en contact de l'élément végétal de *Veratrum* avec une solution aqueuse pendant au moins 10 minutes environ.

8. Procédé selon l'une quelconque des revendications précédentes, dans lequel le solvant comprend un ou plusieurs solvants organiques sélectionnés de préférence dans le groupe constitué de : un alcool organique, un ester, une cétone, un éther, un hydrocarbure halogéné, un hydrocarbure, un aromatique et un hétéroaromatique, et un mélange de deux ou plus de ces solvants.

9. Procédé selon la revendication 8, dans lequel le solvant organique est sélectionné dans le groupe constitué de : méthanol, éthanol, propanol, isopropanol, 2-butanol, n-butanol, acétone, éthyl méthyl cétone, acétate d'éthyle, acétate d'isopropyle, dichlorométhane, dichloroéthane, chloroforme, anisole, benzène, toluéne, xylènes, hexanes, heptanes, pentanes, tétrahydrofurane, dioxane et oxyde de diéthyle, de préférence le méthanol.

10. Procédé selon l'une quelconque des revendications précédentes, dans lequel le solvant comprend un mélange d'un ou plusieurs solvants organiques et d'eau, et/ou une base.

11. Procédé selon la revendication 10, dans lequel le solvant comprend un mélange d'un ou plusieurs solvants organiques et une base sélectionnée dans le groupe comprenant : une solution aqueuse d'hydroxyde d'ammonium, hydroxyde de sodium, carbonate de sodium, bicarbonate de sodium, acétate de sodium, citrate de sodium, hydroxyde de potassium, carbonate de potassium, bicarbonate de potassium, tartrate de sodium et de potassium, et hydroxyde de lithium ; ou dans lequel la base est sélectionnée dans le groupe comprenant : triéthylamine, diéthylisopropylamine et pyridine.

12. Procédé selon la revendication 11, dans lequel le solvant organique est le méthanol.
